Europäisches Patentamt

**European Patent Office**

**Office européen des brevets**

(19)

(11) Publication number: **0 063 870**
**A2**

(12)

# EUROPEAN PATENT APPLICATION

(21) Application number: **82301646.4**

(22) Date of filing: **29.03.82**

(51) Int. Cl.³: **A 61 K 9/06**

(30) Priority: **14.04.81 JP 56494/81**

(43) Date of publication of application: **03.11.82**
**Bulletin 82/44**

(84) Designated Contracting States: **BE DE FR GB IT NL SE**

(71) Applicant: **SUMITOMO CHEMICAL COMPANY, LIMITED,**
**15 Kitahama 5-chome Higashi-ku, Osaka-shi Osaka-fu**
**(JP)**

(72) Inventor: **Yamahira, Yoshiya, 1-11-5, Makami-cho**
**Takatsuki, Osaka (JP)**
Inventor: **Shima, Katsuhiko, 4-10-22, Nakayamate**
**Chuo-ku, Kobe Hyogo (JP)**
Inventor: **Matsusaka, Chikako, 33-3, Maruyama-cho**
**Suita, Osaka (JP)**
Inventor: **Noguchi, Tetsuo, TT10 Brittany Place,**
**Lawrence Kansas 66044 (US)**

(74) Representative: **Allard, Susan Joyce et al, BOULT,**
**WADE & TENNANT 27 Furnival street, London EC4A 1PQ**
**(GB)**

(54) **Cream preparation.**

(57) An antiinflammatory cream preparation which comprises an antiinflammatory substance which is scarcely soluble in water in a base composition ast a concentration of from 0.05 to 1.5% by weight based on the preparation, the base composition comprising a medium chain fatty acid triglyceride, the fatty acid moiety of which contains from 6 to 12 carbon atoms, a carboxyvinyl polymer and purified water, preferably in a wright proportion of 1 - 25 : 0.3 - 3 : 75 - 99, and is adjusted to a pH of 4 to 10. The cream preparation provides good availability of the antiinflammatory substance contained therein and possesses low stimulation and high safety.

CREAM PREPARATION


The present invention relates to a cream preparation. More particularly, it relates to a cream preparation comprising a pharmacologically active substance which is scarcely soluble in water.

For local application, a pharmacologically active substance which is scarcely soluble in water is usually formulated as a solution type preparation such as an ointment in which the active substance is dissolved in a suitable non-aqueous (e.g. propyleneglycol, macrogol) or organic (e.g. ethanol, acetone) solvent so as to enhance its availability. However, a preparation which contains macrogol is sticky and/or stimulates the rectal mucosa. For preparations containing an organic solvent, the application thereof directly to a wound or mucosa is not favourable and occlusive dressing techniques cannot be used.

We have now found that the use of a certain specific base composition affords a suspension type preparation which provides as good an availability of the active ingredient contained therein as for a solution type preparation.

Accordingly, the present invention provides a cream preparation which comprises a pharmacologically active substance which is scarcely soluble in water in a base composition comprising a medium chain fatty acid triglyceride the fatty acid moiety of which contains from 6 to 12 carbon atoms, a carboxyvinyl polymer and purified water, the pH of the cream preparation being within the range of from 4 to 10.

Examples of pharmacologically active substances which may be used in the cream preparations of the invention are steroidal and non-steroidal antiinflammatory compounds such as fluorometholone, prednisolone, dexamethasone, triamcinolone, diflorazone diacetate, indomethacin, ibuprofen and flurubiprofen. The concentration of the active substance is generally from 0.05 to 1.5% by weight based on the preparation

The base composition in the cream preparation comprises a medium chain fatty acid trigylceride, the fatty acid moiety of which contains from 6 to 12 carbon atoms, a carboxyvinyl polymer and purified water.

Examples of suitable fatty acid triglycerides for use in the invention are caproic acid triglyceride, capric acid triglyceride and caprylic-capric acid triglyceride.

The carboxyvinyl polymer is a hydrophilic polymer comprising acrylic acid as the major component thereof. Specific examples are "Carbopol 934", and "Carbopol 941", manufactured by Goodrich Co., Ltd.

To achieve an excellent feel on the use of the composition, good dispersibility and high stability, the weight proportions of the medium chain fatty acid triglyceride, the carboxyvinyl polymer and the purified water are preferably 1-25 : 0.3 - 3 : 75 - 99. The pH of the cream preparation may generally be adjusted to a pH between 4 and 10 by the addition of a pH controlling agent, such as a water-soluble organic amine (e.g. diisopropanolamine).

If desired, other additives may be incorporated into the base composition or the preparation. Examples of such additives are dispersing aids such as glycols (e.g. glycerol, propylene glycol) or sugar fatty acid esters, or absorbing aids such as diisopropyl adipate.

A typical procedure for the production of the cream preparations of the invention comprises dispersing the active substance in an aqueous solution of the carboxyvinyl polymer, adding a water-soluble organic amine thereto to form a gel and adding the medium chain fatty acid triglyceride to the gel for the emulsification thereof.

- 4 -

Alternatively, the water-soluble organic amine may be incorporated after the addition of the medium chain fatty acid triglyceride.

The base of the cream preparation according to this invention has a low stimulation and is very safe. Therefore, it can be applied not only to ordinary skin but also to a mucosa such as the rectum, eye or mouth, and occlusive dressing techniques which are generally used in the field of dermatology are applicable.

The good availability of the active substance in the cream preparation of the invention is illustrated from the results in the following experiment, which shows the transfer rate of indomethacin into muscle.

EXPERIMENT

The availability of indomethacin in the cream preparation obtained as in Example 1 which follows was compared with that of a gel ointment comprising indomethacin in the same concentration as in the said cream preparation ("Inteban ointment" manufactured by Sumitomo Chemical Co., Ltd) and that of an absorptive ointment (Japanese Pharmacopoeia) comprising indomethacin in the same concentration as in the said cream preparation.

Thus, 100 mg of each of the preparations to be tested was applied onto the hair-cut abdomen of a rat within a circle of 3 cm in diameter. After 6 hours, the rat was sacrificed, and the amount of indomethacin in the muscle inside the medicated part was quantitatively determined.

The results are shown in the following table:

Table 1

| Preparation | Type | Concentration of indo-methacin in muscle ($\mu$g/g) |
|---|---|---|
| Example 1 | Suspension | 1.11 ± 0.28 |
| control (Gel ointment) | Solution | 1.05 ± 0.33 |
| Control (Absorptive ointment) | Suspension | 0.57 ± 0.15 |

Note: Average of 4 animals ± SE.

Presently preferred embodiments of the invention

- 6 -

are illustrated in the following Examples.

EXAMPLE 1

To a suspension of indomethacin (1 g) in glycerol (5 g), carboxyvinyl polymer ("Carbopol 940") (1 g) and purified water (92.6 g) were added thereto, followed by stirring to swell the composition. Isopropanolamine (0.4 g) was then added thereto to cause gellation. The resultant mixture was admixed with a medium chain fatty acid triglyceride ("ODO" manufactured by Nisshin Oil Mfg. Co., Ltd.) (3 g) for emulsification to give a cream preparation comprising indomethacin.

EXAMPLE 2

To a mixture of fluorometholone (0.05 g) and a carboxyvinyl polymer ("Carbopol 940") (0.5 g), purified water (83.8 g) was added, followed by stirring to swell the composition. Diisopropanolamine (0.4 g) was added thereto to cause gellation. The resultant mixture was admixed with a medium chain fatty acid triglyceride ("Migriol 810" manufactured by Dynamit Novel A.G.) (15 g) and a sugar fatty acid ester ("DK ester F-160" manufactured by Daiichi Kogyo Seiyaku Co., Ltd) (0.25 g) for emulsification to give a cream preparation comprising fluorometholone.

CLAIMS

1. A cream preparation which comprises a pharmacologically active substance which is scarcely soluble in water in a base composition comprising a medium chain fatty acid triglyceride the fatty acid moiety of which contains from 6 to 12 carbon atoms, a carboxyvinyl polymer and purified water, the pH of the cream preparation being within the range of from 4 to 10.

2. A cream preparation as claimed in Claim 1 wherein the active substance is an antiinflammatory substance.

3. A cream preparation as claimed in Claim 2 wherein the antiinflammatory substance is indomethacin.

4. A cream preparation as claimed in Claim 2 wherein the antiinflammatory substance is fluoromethalone.

5. A cream preparation as claimed in any one of the preceding claims wherein the active substance is contained therein at a concentration of from 0.05 to 1.5% by weight based on the preparation.

0063870

6. A cream preparation as claimed in any one of the preceding claims wherein the base composition contains the fatty acid triglyceride, the carboxyvinyl polymer and the purified water in the weight proportions of 1 - 25 : 0.3 - 3 : 75 - 99.

7. A cream preparation as claimed in any one of the preceding claims wherein the pH of the composition is adjusted by the addition of a pH controlling agent.

SJA/pd/EA572